# EUROPEAN PATENT SPECIFICATION

(11) **EP 1 322 271 B1**
(45) Date of publication and mention of the grant of the patent: **29.06.2011**
(21) Application number: 01970478.2
(22) Date of filing: 28.09.2001
(51) Int. Cl.: A61F 13/475, A61F 13/494

(54) **ABSORBENT ARTICLE WITH LEAKAGE BARRIERS CONTAINING DISTANCE ELEMENTS TO CREATE STABLE TRANSVERSAL CHANNELS**
ABSORBIERENDER ARTIKEL MIT ABSTANDSELEMENTE ENTHALTENDEN LECKAGESPERREN ZUR ERZEUGUNG STABILER QUERKANÄLE
ARTICLE ABSORBANT A BARRIERES DE FUITE CONTENANT DES ELEMENTS DE DISTANCE DESTINES A CREER DES CANAUX TRANSVERSAUX STABLES

(30) Priority: 02.10.2000 SE 0003521
(43) Date of publication of application: 02.07.2003
(73) Proprietor: SCA Hygiene Products AB, 405 03 Göteborg (SE)
(72) Inventor: DREVIK, Solgun, S-435 35 Mölnycke (SE)
(74) Representative: Romare, Laila Anette
(86) International application number: PCT/SE2001/002091
(87) International publication number: WO 2002/028335

(56) References cited:
- EP-A1- 0 649 644
- DE-A1- 4 422 956
- GB-A- 2 284 831
- US-A- 5 667 609

## Description

### TECHNICAL FIELD:

The present invention relates to an absorbent article such as a sanitary napkin. The article comprises an elongate absorbent core delimited by an upper surface and a lower surface, a pair of opposed longitudinal edge portions terminating in longitudinal edges, and a pair of opposed transverse edges. The core has a first end portion, a second end portion and a central portion located between said end portions. A liquid permeable topsheet extends over said upper surface, and a liquid barrier backsheet covers said lower surface of said absorbent core. The absorbent article comprises leakage barriers, each barrier covering a longitudinal edge portion in a respective longitudinal edge portion and forms liquid-retaining pockets along each longitudinal edge portion.

### BACKGROUND OF THE INVENTION:

A common problem associated with an absorbent article such as a diaper or an incontinence pad, which is intended to absorb body fluid, is that fluid leaks out past the side edges of the article. Such leakage is particularly common when gushes of urine are delivered in a short time period and with a high fluid pressure. During urination, the absorbent core cannot instantly absorb all the fluid, which leads to an excessive amount of fluid that flows over the surface of the absorbent article towards the edges not only in the central portion. To help prevent side leakage, it is customary to arrange different types of leakage barriers along the side edges of the article. For example, diapers and incontinence pads are often provided with elastic members which, while the article is being used, are tightened around the user's body and hold the side edges of the article in sealing contact against the body. Elastic members can also be used to form raised edge barriers. It is also possible to create raised barriers in other ways, for example by providing ridges or the like, which prevent liquid from flowing freely over the side edges of the article. Side leakage barriers may also be in the form of a pair of liquid barrier sheets and a pair of strips of resilient material, that forms pockets and increase the stiffness of the sanitary napkin in the region of the mid portion of the absorbent article.

However, it has been found that despite all the efforts, which have hitherto been made to avoid leakage at the side edges, the problem still remains, especially in certain applications. This problem is, for instance, troublesome for bedridden individuals since the risk of leakage increases considerably when the user is lying in a side position. Body fluid which is excreted in this position runs out and gathers, by the effect of gravity, at the longitudinal side edge of the article, where the available absorption material quickly becomes oversaturated with liquid. The remaining liquid that is not absorbed can run freely along the side edge. The risk is of course great that this liquid will be forced out over the side edge of the article and escape if the user moves in such a way that a gap is formed between the article and the user's body.

Conventional hygienic absorbent articles such as sanitary napkins, incontinence pads and the like are provided with an absorbent core which, in theory, is capable of absorbing all the fluid normally discharged by the wearer over an intended exposure time of the article. However, leakage can arise if the absorbent article is not maintained in proper relation with the wearer. One attempt to overcome this problem is to provide a sanitary napkin with so called wings. However, winged sanitary napkins also suffer from certain drawbacks. For example, if a particularly heavy discharge occurs, fluid may spread over the topsheet of the napkin and escape over the wings to thereby stain adjacent clothing. In addition, many wearers regard winged sanitary napkins as being too indiscreet.

Due to their relative narrowness, when sanitary napkins do leak this generally occurs at the side edges. Many attempts have been made to overcome the problem of edge leakage, for example by using strips of resilient material in at least the central portion of the absorbent article which increases the shape stability of the article in the strike zone, i.e. that region of the absorbent article which is first contacted by discharged bodily fluid. In this manner, the risk of bunching of the absorbent article is significantly reduced. In addition, the resilient strips press the longitudinal edges of the absorbent article towards the wearer, thereby causing the article to more readily mould to the body of the wearer. Since the strips need not extend along the entire length of the article, the article may be worn discretely. Advantageously, the remote ends of the strips may serve to impart a bowl-shape to the article to further conform the article to the shape of the wearer.

However, standing gathers is a collective name for these different types of elasticated leakage barriers along the side edges of the article, and they suffer from certain drawbacks. For example, if the standing gathers are subject to an excessive amount of external pressure due to, for example, tight trousers, a soft mattress, when the user is in a vertical position or sitting on a bicycle saddle, the standing gathers will be compressed and thereby somewhat closed, whereby the fluid is prohibited from flowing into the pockets formed by the standing gathers.

Whilst absorbent articles having elasticated side barriers may exhibit improved side edge leakage protection when compared to an absorbent article without elasticated side barriers, a need still exists for an absorbent article which further reduces the risk of side edge leakage whilst still being sufficiently discrete to satisfy the majority of wearers. It is therefore an object of the present invention to provide an absorbent article that meets these requirements.

### SUMMARY OF THE INVENTION:

The present invention relates to an absorbent article such as a sanitary napkin according to the preamble of claim 1, where the absorbent article comprises an elongate absorbent core delimited by an upper surface and a lower surface, a pair of opposed longitudinal edge portions terminating in longitudinal edges, and a pair of opposed transverse edges. Said core has a first end portion, a second end portion and a central portion located between said end portions. A liquid permeable topsheet extends over said upper surface, and a liquid barrier backsheet covers said lower surface of said absorbent core. Said absorbent article comprises leakage barriers, each barrier covering a longitudinal edge portion in a respective longitudinal edge portion and forms liquid-retaining pockets along each longitudinal edge portion

The above-stated objects are achieved in accordance with the present invention by an absorbent article being characterised in that a part of the absorbent article is made profiled and include first spacing means arranged at a distance from each other along the length of the upper surface to create fluid conducting first channels.

The profiled parts of the absorbent article may be the longitudinal edge portions, or the entire upper surface. The profiled parts are made profiled by groove compression of either the topsheet or the upper part of the absorbent core, or the both together, and that the first spacing means consists of the uncompressed parts of the topsheet and/or the upper part of the absorbent core.

Preferred embodiments will become evident by the subsequent dependent claims.

### BRIEF DESCRIPTION OF THE DRAWINGS:

The invention will be described in the following by way of example only and with reference to the attached drawings, in which:
- Fig. 1: is a schematic plan view of an absorbent article according to the present invention;
- Fig. 2: is a sectional view on a larger scale along line II-II of Fig. 1;
- Fig. 3: is a schematic view of a sectional view on a larger scale along line III-III of Fig. 1;
- Fig. 4: is a schematic plan view of an absorbent article according to a second embodiment of the present invention with a partially cut away topsheet; and
- Fig. 5: is a sectional view on a larger scale along line V-V of Fig. 4;
- Fig. 6: is a schematic view of a sectional view on a larger scale along line VI-VI of Fig. 4.
- Fig. 7: is a schematic view of a sectional view on a larger scale along line VI-VI of Fig. 4, according to a further embodiment of the invention.

### DETAILED DESCRIPTION OF PREFERRED EMBODIMENTS:

In the figures, the reference numbers that recur in the different figures refer to the same feature, and additional features in different embodiments are designated new reference numbers.
The invention will be presented in a number of different embodiments and will also refer to previously known sanitary napkins, which constitute examples of different known techniques and shall not be seen as limiting for the present invention. The previously known sanitary napkins constitute examples, on which all the embodiments of the invention may be applicable.

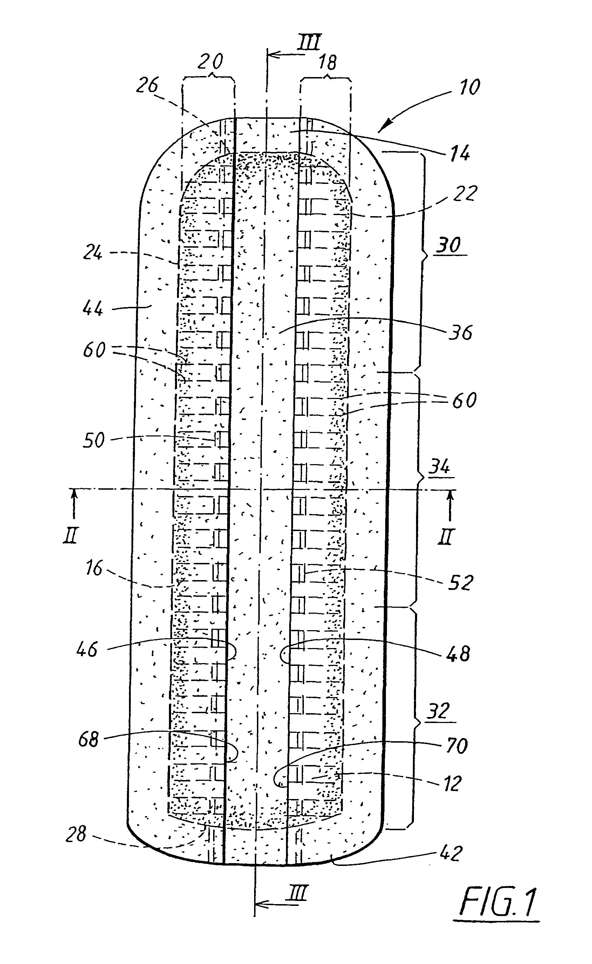

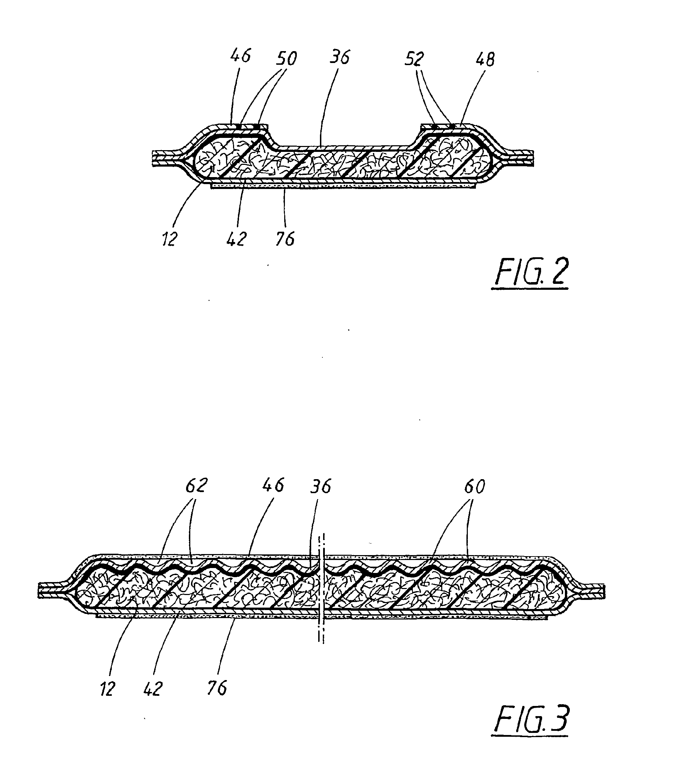

As is apparent from Fig. 1 and 3, reference numeral 10 generally denotes an absorbent article according to the invention. The absorbent article 10 may be a sanitary napkin having an elongate absorbent core 12 delimited by an upper surface 14 and a lower surface 16, and the core 12 may be any conventional absorbent core. The absorbent core further includes opposed longitudinal edge portions 18, 20 terminating in longitudinal edges 22, 24, and a pair of opposed transverse edges 26, 28. The core is made up of a first end portion 30, a second end portion 32 and central portion 34 located between the end portions. In use, the sanitary napkin is intended to be placed relative the wearer so that the strike zone lies within the central portion 34.

As is typical in the art, the absorbent article 10 is further provided with a liquid permeable topsheet 36 extending over the upper surface 14 of the absorbent core 12. The topsheet 36 may be any conventional topsheet. For example, it may be made from a multi-apertured plastics film, or a non-woven material. The absorbent article may also comprise a liquid barrier backsheet 42 extending over the lower surface 16 of the absorbent core 12. The backsheet may be joined to the topsheet 36 to form a peripheral margin 44 around preferably the entire absorbent core 12. On the backsheet 42, there may be fastening means 76 attached for an increased possibility to fasten the absorbent article to the clothing closest to the wearers body. The fastening means may be an adhesive, mechanical fastening means such as Velcro or another fastening means suitable for the purpose.

The sanitary napkin 10 further comprises a pair of two longitudinal leakage barriers 68, 70, each leakage barrier covering a longitudinal edge portion 18, 20 of the permeable topsheet 36, that forms liquid-retaining pockets along each longitudinal edge portion 18, 20.

Fig. 1, 2 and 3 show the invention according to a first embodiment of the invention where a part of the absorbent article 10, such as at least the longitudinal edge portion 18, 20, are made profiled and include first spacing means 60 arranged at a distance from each other along the length of the longitudinal edge portion 18, 20 and will create fluid conducting first channels 62 between the leakage barriers 68, 70 and the top sheet 36 in a direction from the centre of the sanitary napkin 10 to the longitudinal sides of the sanitary napkin 10. The first channels 62 are especially advantageous when the leakage barriers 68, 70 are pressed against the top sheet 36 and the upper surface 14 of the absorbent core 12, by an external force, e.g. tight trousers or if the user is sitting down. The first channels 62 then allows migrating body fluids to flow under the leakage barriers 68, 70 even when the leakage barriers 68, 70 are pressed against the top sheet 36, thereby increasing the flow through the absorbent core 12 rather than through the leakage barriers 68, 70 or over the leakage barriers 68, 70. Since the leakage barriers 68, 70 serve to cover the longitudinal edge portions 18, 20, and to seal the sanitary napkin 10 against the user's body, any transport of fluid towards the side of the sanitary napkin is guided via the first channels 62 into the pockets which are formed beneath the leakage barriers 68, 70 and down through the absorbent core 12, even when the leakage barriers 68, 70 are pressed against the top sheet 36, thereby diminishing the risk of side leakage

The absorbent article according to a second embodiment of the invention is characterized in that each the longitudinal edge portion 18, 20 are made profiled by a groove compression of the topsheet 36. The groove compression compresses the material in certain parts with a distance of 1-15 mm between the uncompressed parts, which also defines the width of the first channels, and that the first spacing means 60 consists of the uncompressed parts.

The absorbent article according to a third embodiment of the invention is characterized in that the longitudinal edge portion 18, 20 also may be made profiled by a groove compression of the upper part of the absorbent core 12, and the first spacing means 60 consists of the uncompressed parts. According to a fourth embodiment of the invention, the entire upper surface 14 are made profiled by a groove compression of the topsheet 36, and the first spacing means 60 consists of the uncompressed parts.

According to a fifth embodiment of the invention, the entire upper surface 14 are made profiled by a groove compression of an upper part of the absorbent core 12, and that the first spacing means 60 consists of the uncompressed parts.

According to a sixth embodiment of the invention, the profiled parts are made profiled by groove compression of both the topsheet 36 and the upper part of the absorbent core 12, and that the first spacing means 60 consists of the uncompressed parts of the topsheet 36 and the upper part of the absorbent core 12.

According to a seventh embodiment of the invention, a distance material in the form of cylinders are placed in at least the first area, in a direction from the centre of the sanitary napkin 10 to the longitudinal sides of the sanitary napkin 10, and that the first spacing means 60 consists of the distance material.

All the above mentioned embodiments, one to seven, together with the embodiments that will be described below, will form a number of further embodiments. It is to be understood that all combinations conceivable form new embodiments of the invention.

Figures 1, 2 and 3 may be further used as a support in the description of yet further embodiments of the invention, where the longitudinal leakage barriers 68, 70 are in the form of barrier strips 46, 48, with each sheet covering a longitudinal edge portion 18, 20 of the permeable topsheet 36, that forms pockets between the top sheet 36 and the barrier strips 46, 48 along the side edges. The barrier strips 46, 48 may be of either a liquid barrier material or a material that at least resists fluid penetration, e.g. a non-woven hydrophobic fibrous web or another material suitable for the purpose. It is an advantage if the barrier material is breathable, i.e. will permit the passage of air and water vapour. Since the barrier strips 46, 48 may be joined with the peripheral margin 44, the pockets along the edge portions 18, 20 may be seen as enclosed by the barrier strips 46, 48 and the topsheet 36. A pair of pretensioned, longitudinal elastic members 50, 52 are arranged along the barrier strips 46, 48 of the sanitary napkin 10. The elastic members 50, 52 are designed to curve the sanitary napkin 10 to the shape of the user's body and at the same time they constitute means for raising the barrier strips 46, 48 from the upper surface 14 of the sanitary napkin 10. Thus, the elastic members 50, 52 serve to hold the barrier strips 46, 48 of the sanitary napkin 10 in contact against the user's body, in order to ensure that, during use, no gap arises between the sanitary napkin 10 and the user's body, through which gap body fluid could leak from the sanitary napkin 10. Since the material of the barrier strips 46, 48 is primarily hydrophobic, migrating body fluids tend to flow through the absorbent core rather than through the resilient barrier strips 46, 48. Since the barrier strips 46, 48 serve to conceal the longitudinal edge portions 18, 20, any collection of fluid at the edge portions is concealed, thereby imparting an impression of increased safety and cleanliness to the wearer.

Advantageously, the elastic members 50, 52 are made of a three dimensional elastic material such as an elastic polymer, elastic non-woven fibrous plastic, elastic foam, silicone, rubber or another material suitable for the purpose.

The elastic members 50, 52 may be in the form of a string of beads (not shown). The beads serve as second spacing means between the barrier strips 46, 48 and the top sheet 36, and will create fluid conducting second channels (not shown) between the barrier strips 46, 48 and the top sheet 36 in a direction from the centre of the sanitary napkin 10 to the longitudinal sides of the sanitary napkin 10. The second channels 62 are especially advantageous when the barrier strips 46, 48 are pressed against the top sheet 36 and the upper surface 14 of the absorbent core 12, by an external force, e.g. tight trousers or if the user is sitting down. The second channels then allows migrating body fluids to flow under the barrier strips 46, 48 even when the barrier strips 46, 48 are pressed against the top sheet 36, thereby increasing the flow through the absorbent core 12 rather than through the barrier strips 46, 48 or over the barrier strips 46, 48. Since the barrier strips 46, 48 serve to cover the longitudinal edge portions 18, 20, and to seal the sanitary napkin 10 against the user's body, any transport of fluid towards the side of the sanitary napkin is guided via the second channels into the pockets which are formed beneath the barrier strips 46, 48 and down through the absorbent core 12, even when the barrier strips 46, 48 are pressed against the top sheet 36, thereby diminishing the risk of side leakage.

The second spacing means together with the first spacing means will advantageously create channels defined by the combined space of the second channels and the first channels, which means that all of the above mentioned advantages with distance material creating channels will be enhanced.

The beads may alternatively have other cross-sectional shapes, such as an oval shape. The beads may also have different diameters and/or different cross sections in the same string of beads.

The string of beads may be created by, for example, point sealing of a three dimensional elastic thread such as an elastic polymer, elastic non-woven fibrous plastic, elastic foam, silicone, rubber or another material suitable for the purpose or two elastic webs with non-elastic spacers between.

The elastic members 50, 52 may be broader than in the first embodiment and they are in the form of a string of short cylinders (not shown). The short cylinders serve as second spacing means between the barrier strips 46, 48 and the top sheet 36, and will create second channels between the barrier strips 46, 48 and the top sheet 36 in a direction from the centre of the sanitary napkin 10 to the longitudinal sides of the sanitary napkin 10, and serve the same purpose as in the first embodiment. Here, short cylinder means a cylinder having a length less than the cylinder diameter. The cylinder may alternatively have other cross-sectional shapes, such as an oval shape. The cylinders may also have different diameters and/or different cross sections in the same string of cylinders.

The string of short cylinders may be created by, for example, point sealing of a three dimensional elastic thread such as an elastic polymer, elastic non-woven fibrous plastic, elastic foam, silicone, rubber or another material suitable for the purpose or two elastic webs with non-elastic spacers between.

The elastic members 50, 52 may be even broader than in the second embodiment and they are in the form of a string of long cylinders (not shown). The long cylinders serve as second spacing means 60 between the barrier strips 46, 48 and the top sheet 36, and will create second channels between the barrier strips 46, 48 and the top sheet 36 in a direction from the centre of the sanitary napkin 10 to the longitudinal sides of the sanitary napkin 10, and serves the same purpose as in the second embodiment. Here, long cylinder means a cylinder length longer than the cylinder diameter. The cylinder may alternatively have other cross-sectional shapes, such as an oval shape. The cylinders may also have different diameters and/or different cross sections in the same string of cylinders.

The string of long cylinders may be created by, for example, point sealing of a three dimensional elastic thread such as an elastic polymer, elastic non-woven fibrous plastic, elastic foam, silicone, rubber or another material suitable for the purpose or two elastic webs with non-elastic spacers between.

The string of long cylinders may be substituted by a distance material (not shown) in the form of long cylinders and elastic members, e.g. the elastic members 50, 52 previously mentioned. The cylindrical distance material is placed within the barrier strips 46, 48 or in a manner suitable for the purpose of using the distance material to create second channels between the barrier strips 46, 48 and the top sheet 36 in a direction from the centre of the sanitary napkin 10 to the longitudinal sides of the sanitary napkin 10. The elastic members 50, 52 may be a pair of pretensioned, longitudinal elastic members 50, 52 that are arranged along the barrier strips 46, 48 of the sanitary napkin 10. The elastic members 50, 52 are designed to curve the sanitary napkin 10 to the shape of the user's body and at the same time they constitute means for keeping open the side pockets created beneath the barrier strips 46, 48 and the sanitary napkin 10.

The distance material in the form of long cylinders according to the fourth embodiment of the invention may alternatively have other cross-sectional shapes, such as an oval shape. The distance material may also have different diameters and/or different cross sections in the barrier strips 46, 48.

The long cylinders may be a three dimensional elastic thread such as an elastic polymer, elastic non-woven fibrous plastic, elastic foam, silicone, rubber or another material suitable for the purpose or two elastic webs with non-elastic spacers between.

The short and the long cylinders may be up to 50 mm long.

The distance between the second spacing means, i.e. the width of the second channel, is 1-20 mm.

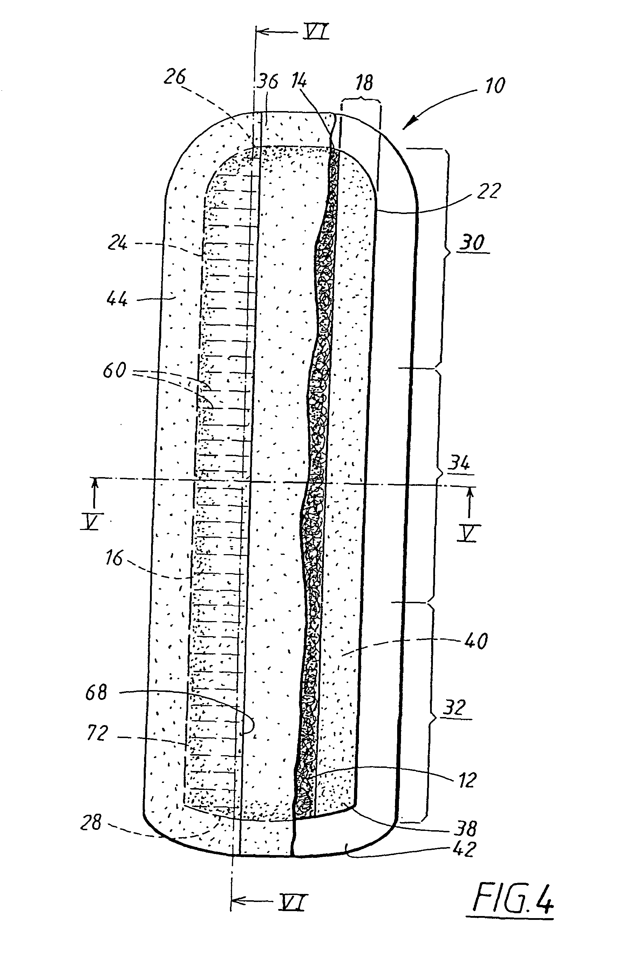

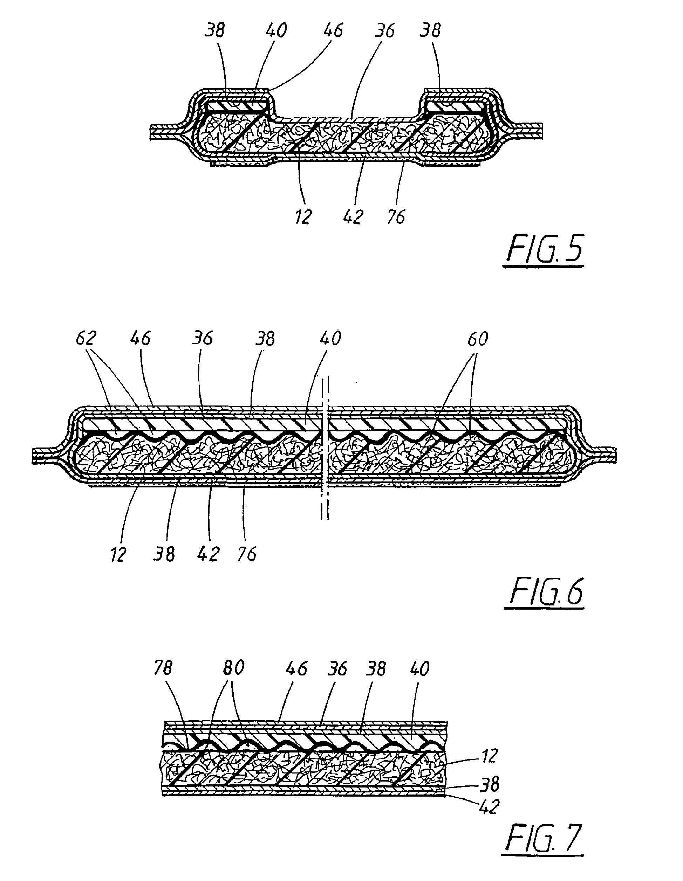

Figures 4, 5 and 6 show yet further embodiments of the present invention, where the above mentioned first embodiment to sixth embodiment of the invention are to be combined with a previously known sanitary napkin and as is typical in the art, the absorbent article 10 comprises leakage barriers 68 in the form of a pair of liquid barrier sheets 38 and a pair of strips of resilient material 40, with each liquid barrier sheet 38 covering a longitudinal edge portion 18, 20 of the permeable topsheet 36. The strips of resilient material 40 may be placed either over or under the top sheet, but in the figures the strips of resilient material 40 is placed under the top sheet 36, which also will be described in the following embodiments. The liquid barrier sheets 38 may be placed either over or under the top sheet, but in the figures the strips of liquid barrier sheets 38 is placed over the top sheet 36, which also will be described in the following embodiments. The liquid barrier sheets are preferably coated with adhesive on their concave surface, i.e. the surface facing towards the absorbent body. Although a pair of separate barrier sheets has been shown in the drawings, it is to be realized, however, that the two sheets 38 may be replaced by a single sheet extending over the entire lower surface 16 of the absorbent core, being folded over the longitudinal edges 22, 24 and covering the longitudinal edge portions 18, 20 of the permeable top sheet 36.

In this previously known sanitary napkin it is also known, and as best illustrated in Fig. 4, 5 and 6, that the strip of resilient material 40 is placed between each liquid impermeable sheet 38 and the absorbent core 12 along each longitudinal edge portion 18, 20 of the absorbent core in at least the mid portion 34 of the absorbent core. The strips of resilient material serve to significantly increase the stiffness of the sanitary napkin in the region of the mid portion. In other words, the resistance to flexure in the longitudinal direction is increased by at least 10% in the longitudinal edge portions in the mid portion when compared to a sanitary napkin without strips of resilient material.

Advantageously, the strips of resilient material 40 are primarily hydrophobic and may be a three dimensional non-woven fibrous plastic wadding or a foamed plastic. Since the material of the strips is primarily hydrophobic, migrating body fluids tend to flow through the absorbent core rather than through the strips of resilient material. Since, in the embodiment shown in Fig. 2, the strips of resilient material serve to conceal the longitudinal edge portions 18, 20 in the mid portion 34, any collection of fluid at the edge portions is concealed, thereby imparting an impression of increased safety to the wearer.

According to another embodiment of the invention the strips of resilient material 40 may also be profiled by groove compression, on that surface that faces the upper side of the absorbent core 12. Thus, the strips of resilient material 40 include third spacing means 78 in the form of uncompressed resilient material arranged at a distance from each other along the length of the resilient material 40 to create third fluid conducting third channels 80 between the strips of resilient material 40 and the absorbent core 12, and the third spacing means 78 consists of the uncompressed parts of the resilient material 40.

In a further embodiment of the previously known sanitary napkin, strips of resilient material 40 are placed between the liquid impermeable sheets 38 and the lower surface 16 of the absorbent core 12. Preferably, however, the strips of resilient material are placed between the liquid impermeable sheets 38 and the upper surface 14 of the absorbent core. In this manner, the strips of resilient material 40 serve to "lift" the liquid impermeable sheets 38 from the upper surface 14 of the absorbent core to thereby create an opening to a liquid-retaining pocket formed by the liquid impermeable sheet along each longitudinal edge portion 18, 20. Accordingly, any discharged bodily fluids which migrate across the upper surface of the absorbent core will flow through the opening and into the liquid-retaining pocket. Due to the fact that the concave surfaces of the sheets of resilient material are coated with adhesive and thereby adhere to the absorbent core and strips of resilient material, the risk of fluids leaking from the liquid-retaining pockets is minimal.

Particularly in the case when the absorbent article comprises a pair of liquid impermeable sheets 38 covering the longitudinal edge portions 18, 20 of the absorbent core 12, the absorbent article may also comprise a liquid impermeable backsheet 42 extending over the lower surface 16 of the absorbent core. Advantageously, the backsheet 42 is made of the same material as the liquid impermeable sheet 38 and is joined to the topsheet 36 to form a peripheral margin 44 around preferably the entire absorbent core 12. In this manner, the liquid-retaining pockets along the edge portions 18, 20 are enclosed by the topsheet 36 and the backsheet 42.

For ease of manufacturing and to ensure that the risk of edge leakage anywhere along the length of the absorbent article is minimal, the liquid retaining pockets preferably extend along the entire length of the absorbent core 12. Since, however, the form stability of the absorbent article is most critical only in the mid portion 34 of the absorbent core, the strips of resilient material 40 need only occupy said mid portion. However, if desired, the strips may extend into the first and second end portions 30, 32.
The pockets may extend along the entire length of said absorbent core 12, or extend along the central portion 34 of said absorbent core 12.

The absorbent article according to any one of the preceding embodiments is characterized in that the thickness of the first, second and third spacing means is at least 1 mm, and that the sum of the thickness of the first and second spacing means or the first and third spacing means or the second and the third spacing means defines the respective combination's maximum height of the sum of the first channel and/or second channel and/or third channel. The thickness of the spacing means also means the diameter when the spacing means are cylindrical or spherical.

The distance between the third spacing means 78, i.e. the width of the third channels, is 1-15 mm.

Advantageously, the mid portion 34 of the absorbent core 12 comprises between 20% and 60%, preferably between 30 and 45%, and most preferably about one third, of the length of the absorbent core.

Obviously, the hydrophobic strips of resilient material 40 or the barrier strips 46, 48, i.e. the leakage barriers 68, 70 may not extend over the entire width of the absorbent core since this would prevent passage of body fluids into the absorbent core 12. Accordingly, the leakage barriers 68, 70 have an extension transversely across the absorbent core, with the extension being between 5% and 25% of the total width of the sanitary napkin at the wetting area. The liquid impermeable sheets 38 advantageously display the same or similar transverse extension as the strips of resilient material. The thickness of the strips of resilient material 40, i.e. the extension of the strips in a direction substantially perpendicular to the upper surface of the absorbent core, should be sufficient to allow the strips to mould to the wearer's body. Typically, the thickness of the strips in an uncompressed condition can thus lie between about 1 and 5 mm.
The invention is not restricted to the embodiments described above and shown in the drawings, but may be modified within the scope of the appended claims. For example, strips of resilient material may be placed on both surfaces of the absorbent core in the longitudinal edge portions.

The absorbent article may be a sanitary napkin, a diaper, an incontinent protection article, a panty liner or any absorbent article suitable for the purpose. The absorbent article is not restricted to any shape or configuration, but may be of any shape suitable for the purpose, e.g. triangular, hour-glass shaped or rectangular. Also, the elastic members and the distance material may be of a stiff material, and the side barriers with the distance material will still create the channels for transporting the fluids. The sanitary napkin may also be equipped with wings.

In making a sanitary napkin in another shape than the one shown in the figures, advantageously the topsheet 36 will not extend outside the absorbent core, and the backsheet 42 or the liquid impermeable sheet 38 will not toghether with the topsheet 36 form a peripheral margin 44, but the backsheet 42 or the liquid impermeable sheet 38 will be folded over the side edges over the topsheet. The barrier strips 46, 48, in the embodiments referring to fig 4, 5, 6 or 7 may be excluded, and shall only be seen as a possibility to reinforce and/or modify the sanitary napkin according to certain needs.

The elastic members not only serve as means for creating channels, but may also bend the sanitary napkin into a cup-shape with the whetting area as one of the low points.

There may also be a combination of the above-described embodiments of the invention. For instance, the resilient material 40 may be profiled at the same time as the absorbent core, together creating fluid conducting channels.

## Claims

1. An absorbent article (10) such as a sanitary napkin, said article comprising:
an elongate absorbent core (12) delimited by an upper surface (14) and a lower surface (16), a pair of opposed longitudinal edge portions (18, 20) terminating in longitudinal edges (22, 24), and a pair of opposed transverse edges (26, 28), said core having a first end portion (30), a second end portion (32) and a central portion (34) located between said end portions;
a liquid permeable topsheet (36) extending over said upper surface (14), and
a liquid barrier backsheet (42) covering said lower surface (16) of said absorbent core (12),
leakage barriers (68, 70), each barrier covering a longitudinal edge portion (18, 20) in a respective longitudinal edge portion (18, 20) and forming liquid-retaining pockets along each longitudinal edge portion,
**characterized in, that** a part of the absorbent article is made profiled and include first spacing means (60), where the first spacing means (60) are arranged between the absorbent core (12) and each of said leakage barriers (68, 70), said first spacing means being arranged at a distance from each other along the length of said leakage barriers (68, 70) and defining fluid conducting first channels (62), extending generally perpendicularly to the longitudinal edge portions (18, 20), between said leakage barriers (68, 70) and said absorbent core (12).

2. The absorbent article according to claims 1,
**characterized in, that** each longitudinal edge portion (18, 20) is made profiled.

3. The absorbent article according to claim 1,
**characterized in, that** the upper surface (14) is made profiled.

4. The absorbent article according to claim 2 or 3,
**characterized in, that** the profiled parts are created by groove compression of the top sheet (36), and that the first spacing means (60) consists of the uncompressed parts of the top sheet (36).

5. The absorbent article according to claim 2 or 3 ,
**characterized in, that** the profiled parts are made profiled by groove compression of the upper part of the absorbent core (12), and that the first spacing means (60) consists of the uncompressed parts of the absorbent core (12).

6. The absorbent article according to claim 2 or 3,
**characterized in, that** the profiled parts are made profiled by groove compression of both the topsheet (36) and the upper part of the absorbent core (12), and that the first spacing means (60) consists of the uncompressed parts of the topsheet (36) and the upper part of the absorbent core (12).

7. The absorbent article according to claim 2 or 3,
**characterized in, that** the profiled parts are made profiled by a distance material in the form of cylinders, that are placed in the topsheet (36) of each profiled portion, in a direction from the centre of the sanitary napkin (10) to the longitudinal sides of the sanitary napkin 10, and that the first spacing means (60) consists of the distance material.

8. The absorbent article according to claim 2 or 3,
**characterized in, that** the profiled parts are made profiled by a distance material in the form of cylinders, that are placed in the upper part of the absorbent core (12) of each profiled portion, in a direction from the centre of the sanitary napkin (10) to the longitudinal sides of the sanitary napkin 10, and that the first spacing means (60) consists of the distance material.

9. The absorbent article according to any one of the preceding claims,
**characterized in, that** the leakage barriers (68, 70) consists of barrier strips (46, 48), each strip covering a respective longitudinal edge portion (18, 20) and forming liquid-retaining pockets along each longitudinal edge portion, a pair of longitudinal elastic members (50, 52) are arranged along said barrier strips (46, 48) that are placed along each longitudinal edge portion (18, 20) of said absorbent core in at least said central portion (34) of said absorbent core

10. The absorbent article according to claim 9,
**characterized in, that** the elastic members (50, 52) is in the shape of a string of beads, and that the beads defines second spacing means.

11. The absorbent article According to claim 9,
**characterized in, that** the elastic members (50, 52) is in the shape of a string of short cylinders having a length less than the cylinder diameter, and that the short cylinders defines second spacing means.

12. The absorbent article according to claim 9,
**characterized in, that** the elastic members (50, 52) is in the shape of a string of long cylinders having a length longer than the cylinder diameter, and that the long cylinders defines second spacing means.

13. The absorbent article according to claim 9,
**characterized in, that** a distance material in the form of long cylinders are placed within the barrier strips 46, 48 in a direction from the centre of the sanitary napkin 10 to the longitudinal sides of the sanitary napkin 10.

14. The absorbent article according to any one of the preceding claims, **characterized in, that** said barrier strips (46, 48) is primarily hydrophobic.

15. The absorbent article according to any one of the preceding claims, **characterized in, that** said side elastic members (50, 52) is a three dimensional non-woven fibrous plastic wadding or a foamed plastic.

16. The absorbent article according to any one of the claims 1 to 8, **characterized in, that** a liquid impermeable sheet (38) covering said longitudinal edge portions (18, 20) of said absorbent core (12) and that the leakage barriers (68, 70) consists of a strip of resilient material (40) that is placed between said liquid impermeable sheet (38) and said absorbent core (12) along each longitudinal edge portion (18, 20) of said absorbent core in at least said mid portion (34) of said absorbent core to thereby increase the flexure resistance of the article.

17. The absorbent article according to claim 16,
**characterized in, that** said strip of resilient material (40) is a three dimensional nonwoven fibrous plastic wadding or a foamed plastic.

18. The absorbent article according to claims 16 or 17,
**characterized in, that** said strip of resilient material (40) is placed between said liquid impermeable sheet (38) and said lower surface (16) of said absorbent core (12).

19. The absorbent article according to any one of the claims 16 to 18, **characterized in, that** it comprises a pair of liquid impermeable sheets (38), each sheet covering a respective longitudinal edge portion (18, 20) and forming liquid-retaining pockets along each longitudinal edge portion (18, 20).

20. The absorbent article according to any one of the preceding claims, **characterized in, that** the side of said strip of resilient material (40) that faces the upper side of the absorbent core 12 is made profiled by groove compression.

21. The absorbent article according to claim 20, **characterized in, that** the uncompressed profiled parts of the strip of resilient material (40) defines third spacing means (78)

22. The absorbent article according to claim 19, **characterized in, that** it comprises a liquid impermeable backsheet (42) extending over said lower surface (16) of said absorbent core, said backsheet (42) and said topsheet (36) being joined to form a peripheral margin (44) around said absorbent core to thereby enclose said liquid-retaining pockets along said edge portions.

23. The absorbent article according to any one of the preceding claims, **characterized in, that** said pockets extend along the entire length of said absorbent core (12).

24. The absorbent article according to any one of the preceding claims, **characterized in, that** said pockets extend along the central portion (34) of said absorbent core (12).

25. The absorbent article according to any one of the preceding claims, **characterized in, that** said central portion (34) of said absorbent core comprises between 20% and 60%, preferably between 30 and 45%, and most preferably about one third, of the length of the absorbent core (12).

26. The absorbent article according to any one of the preceding claims, **characterized in, that** said leakage barriers (68, 70) each have an extension transversely across said absorbent core (12), said extension being between 5% and 25% of the total width of the sanitary napkin at the wetting area.

27. The absorbent article according to any one of the preceding claims, **characterized in, that** the thickness of the first, second and third spacing means is at least 1 mm.

## Patentansprüche

1. Absorptionsartikel (10), wie zum Beispiel eine Binde, wobei der Artikel umfasst:
einen länglichen Absorptionskern (12), der durch eine obere Oberfläche (14) und eine untere Oberfläche (15), ein Paar einander gegenüberliegender längsseitiger Kantenabschnitte (18, 20), die in längsseitigen Kanten (22, 24) enden, und ein Paar einander gegenüberliegender Querkanten (26, 28) begrenzt ist, wobei der Kern einen ersten Endabschnitt (30), einen zweiten Endabschnitt (32) und einen zentralen Abschnitt (34), der zwischen den Endabschnitten angeordnet ist, aufweist,
eine flüssigkeitsdurchlässige Oberlage (36), die sich über die obere Oberfläche (14) erstreckt, und
eine Flüssigkeitsbarrierenrücklage (42), die die untere Oberfläche (16) des Absorptionskerns (12) bedeckt,
Leckagebarrieren (68, 70), wobei jede Barriere einen längsseitigen Kantenabschnitt (18, 20) in einem entsprechenden längsseitigen Kantenabschnitt (18, 20) bedeckt und Flüssigkeit aufnehmende Taschen entlang jedes längsseitigen Kantenabschnitts bildet,
**dadurch gekennzeichnet, dass** ein Teil des Absorptionsartikels mit Profil versehen ist und erste Abstandsmittel (60) enthält, wobei die ersten Abstandsmittel (60) zwischen dem Absorptionskern (12) und jeder der Leckagebarrieren (68, 70) angeordnet sind, wobei die ersten Abstandsmittel in einem Abstand voneinander entlang der Länge der Leckagebarrieren (68, 70) angeordnet sind und Fluid führende erste Kanäle (62) defiinieren, die sich allgemein senkrecht zu den längsseitigen Kantenabschnitten (18, 20) zwischen den Leckagebarrieren (68, 70) und dem Absorptionskern (12) erstrecken.

2. Absorptionsartikel nach Anspruch 1,
**dadurch gekennzeichnet, dass** jeder längsseitige Kantenabschnitt (18, 20) mit einem Profil versehen ist.

3. Absorptionsartikel nach Anspruch 1,
**dadurch gekennzeichnet, dass** die obere Oberfläche (14) mit einem Profil versehen ist.

4. Absorptionsartikel nach Anspruch 2 oder 3,
**dadurch gekennzeichnet, dass** die mit Profil versehenen Teile durch eine Nutkompression der Oberlage (36) erzeugt werden und dass die ersten Abstandsmittel (60) aus unkomprimierten Teilen der Oberlage (36) bestehen.

5. Absorptionsartikel nach Anspruch 2 oder 3,
**dadurch gekennzeichnet, dass** die mit Profil versehenen Teile durch Nutkompression des oberen Teils des Absorptionskerns (12) mit dem Profil versehen sind und dass die ersten Abstandsmittel (60) aus den unkomprimierten Teilen des Absorptionskerns (12) bestehen.

6. Absorptionsartikel nach Anspruch 2 oder 3,
**dadurch gekennzeichnet, dass** die mit Profil versehenen Teile durch Nutkompression sowohl der Oberlage (36) als auch des oberen Teils des Absorptionskerns (12) mit dem Profil versehen werden und dass die ersten Abstandsmittel (60) aus den unkomprimierten Teilen der Oberlage (36) und des oberen Teils des Absorptionskerns (12) bestehen.

7. Absorptionsartikel nach Anspruch 2 oder 3,
**dadurch gekennzeichnet, dass** die mit Profil versehenen Teile durch ein Abstandsmaterial in der Form von Zylindern, die in der Oberlage (36) jedes mit Profil versehenen Abschnitts platziert sind, in einer Richtung von der Mitte der Binde (10) zu den längsseitigen Seiten der Binde 10 mit dem Profil versehen sind und dass die ersten Abstandsmittel (60) aus dem Abstandsmaterial bestehen.

8. Absorptionsartikel nach Anspruch 2 oder 3,
**dadurch gekennzeichnet, dass** die mit Profil versehenen Teile durch ein Abstandsmaterial in der Form von Zylindern, die in dem oberen Teil des Absorptionskerns (12) jedes mit Profil versehenen Abschnitts platziert sind, in einer Richtung von der Mitte der Binde (10) zu den längsseitigen Seiten der Binde 10 mit dem Profil versehen sind und dass die ersten Abstandsmittel (60) aus dem Abstandsmaterial bestehen.

9. Absorptionsartikel nach einem der vorhergehenden Ansprüche,
**dadurch gekennzeichnet, dass** die Leckagebarrieren (68, 70) aus Barrierestreifen (46, 48) bestehen, wobei jeder Streifen jeweils einen längsseitigen Kantenabschnitt (18, 20) bedeckt und Flüssigkeit enthaltende Taschen entlang jedes längsseitigen Kantenabschnitts bildet, wobei ein Paar längsseitiger elastischer Elemente (50, 52) entlang der Barrierestreifen (46, 48), die entlang jedes längsseitigen Kantenabschnitts (18, 20) des Absorptionskerns zumindest in dem Mittelabschnitt (34) des Absorptionskerns platziert sind, angeordnet ist.

10. Absorptionsartikel nach Anspruch 9,
**dadurch gekennzeichnet, dass** die elastischen Elemente (50, 52) in der Form einer Perlenkette sind und dass die Perlen zweite Abstandsmittel definieren.

11. Absorptionsartikel nach Anspruch 9,
**dadurch gekennzeichnet, dass** die elastischen Elemente (50, 52) in der Form eines Strangs kurzer Zylinder mit einer kürzeren Länge als dem Zylinderdurchmesser sind und dass die kurzen Zylinder zweite Abstandsmittel definieren.

12. Absorptionsartikel nach Anspruch 9,
**dadurch gekennzeichnet, dass** die elastischen Elemente (50, 52) in der Form eines Strangs langer Zylinder mit einer längeren Länge als dem Zylinderdurchmesser sind und dass die langen Zylinder zweite Abstandsmittel definieren.

13. Absorptionsartikel nach Anspruch 9,
**dadurch gekennzeichnet, dass** ein Abstandsmaterial in der Form langer Zylinder innerhalb der Barrierestreifen 46, 48 in einer Richtung von der Mitte der Binde 10 zu den längsseitigen Seiten der Binde 10 platziert ist.

14. Absorptionsartikel nach einem der vorhergehenden Ansprüche, **dadurch gekennzeichnet, dass** die Barrierestreifen (46, 48) primär hydrophob sind.

15. Absorptionsartikel nach einem der vorhergehenden Ansprüche, **dadurch gekennzeichnet, dass** die seitlichen elastischen Elemente (50, 52) ein dreidimensionales Vlies-Faser-Kunststoff-Füllmaterial oder ein Kunststoffschaum sind.

16. Absorptionsartikel nach einem der Ansprüche 1 bis 8, **dadurch gekennzeichnet, dass** eine flüssigkeitsundurchlässige Lage (38) die längsseitigen Kantenabschnitte (18, 20) des Absorptionskerns (12) bedeckt und dass die Leckagebarrieren (68, 70) aus einem Streifen aus elastischem Material (40) bestehen, der zwischen der flüssigkeitsundurchlässigen Lage (38) und dem Absorptionskern (12) entlang jedes längsseitigen Kantenabschnitts (18, 20) des Absorptionskerns zumindest in dem Mittelabschnitt (34) des Absorptionskerns platziert ist, um dadurch die Biegebeständigkeit des Artikels zu erhöhen.

17. Absorptionsartikel nach Anspruch 16,
**dadurch gekennzeichnet, dass** der Streifen elastischen Materials (40) ein dreidimensionales Vlies-Faser-Kunststoff-Füllmaterial oder ein Kunststoffschaum ist.

18. Absorptionsartikel nach Anspruch 16 oder 17,
**dadurch gekennzeichnet, dass** der Streifen elastischen Materials (40) zwischen der flüssigkeitsundurchlässigen Lage (38) und der unteren Oberfläche (16) des Absorptionskerns (12) platziert ist.

19. Absorptionsartikel nach einem der Ansprüche 16 bis 18, **dadurch gekennzeichnet, dass** er ein Paar flüssigkeitsundurchlässiger Lagen (38) aufweist, wobei jede Lage jeweils einen längsseitigen Kantenabschnitt (18, 20) bedeckt und Flüssigkeit enthaltende Taschen entlang des jeweiligen längsseitigen Kantenabschnitts (18, 20) bildet.

20. Absorptionsartikel nach einem der vorhergehenden Ansprüche, **dadurch gekennzeichnet, dass** die Seite des Streifens elastischen Materials (40), die der oberen Seite des Absorptionskerns 12 zugewandt ist, durch Nutkompression mit einem Profil versehen ist.

21. Absorptionsartikel nach Anspruch 20, **dadurch gekennzeichnet, dass** die unkomprimierten, mit Profil versehenen Teile des Streifens elastischen Materials (40) dritte Abstandsmittel (78) definieren.

22. Absorptionsartikel nach Anspruch 19,
**dadurch gekennzeichnet, dass** er eine flüssigkeitsundurchlässige Rücklage (42) aufweist, die sich über die untere Oberfläche (16) des Absorptionskerns erstreckt, wobei die Rücklage (42) und die Oberlage (36) verbunden sind, um einen umfangsseitigen Rand (44) um den Absorptionskern zu bilden, um dadurch die Flüssigkeit enthaltenden Taschen entlang der Kantenabschnitte einzuschließen.

23. Absorptionsartikel nach einem der vorhergehenden Ansprüche, **dadurch gekennzeichnet, dass** sich die Taschen entlang der gesamten Länge des Absorptionskerns (12) erstrecken.

24. Absorptionsartikel nach einem der vorhergehenden Ansprüche, **dadurch gekennzeichnet, dass** sich die Taschen entlang des Mittelabschnitts (34) des Absorptionskerns (12) erstrecken.

25. Absorptionsartikel nach einem der vorhergehenden Ansprüche, **dadurch gekennzeichnet, dass** der Mittelabschnitt (34) des Absorptionskerns zwischen 20% und 60%, bevorzugt zwischen 30 und 45% und am meisten bevorzugt etwa ein Drittel, der Länge des Absorptionskerns (12) umfasst.

26. Absorptionsartikel nach einem der vorhergehenden Ansprüche, **dadurch gekennzeichnet, dass** die Leckagebarrieren (68, 70) jeweils eine Ausdehnung quer über den Absorptionskern (12) haben, wobei die Ausdehnung zwischen 5% und 25% der gesamten Breite der Binde in dem Feuchtigkeitsbereich liegt.

27. Absorptionsartikel nach einem der vorhergehenden Ansprüche, **dadurch gekennzeichnet, dass** die Dicke des ersten, zweiten und dritten Abstandsmittels mindestens 1 mm beträgt.

## Revendications

1. Article absorbant (10) tel qu'une serviette hygiénique, ledit article comprenant:
un coeur absorbant de forme allongée (12) délimité par une surface supérieure (14) et une surface inférieure (15), une paire de parties de bords longitudinaux opposées (18, 20) se terminant en bords longitudinaux (22, 24), et une paire de bords transversaux opposés (26, 28), ledit coeur ayant une première partie d'extrémité (30), une deuxième partie d'extrémité (32) et une partie centrale (34) située entre lesdites parties d'extrémité;
une feuille supérieure (35) perméable aux liquides s'étendant sur ladite surface supérieure (14), et
une feuille inférieure (42) imperméable aux liquides recouvrant ladite surface inférieure (16) du coeur absorbant (12),
des barrières anti-fuite (68, 70), chaque barrière recouvrant une partie de bord longitudinal (18, 20) dans une partie de bord longitudinal (18, 20) respective et formant des poches de retenue de liquide le long de chaque partie de bord longitudinal,
**caractérisé en ce que** une partie de l'article absorbant est profilée et comprend des premiers moyens d'espacement (60), les premiers moyens d'espacement (60) étant placés entre le coeur absorbant (12) et chacune desdites barrières anti-fuite (68, 70), lesdits premiers moyens d'espacement (60) étant placés à distance les uns des autres le long de la longueur desdites barrières anti-fuite (68, 70) et définissant des premiers canaux (62) conduisant les fluides, s'étendant de façon générale perpendiculairement aux parties de bords longitudinaux (18, 20), entre lesdites barrières anti-fuite (68, 70) et ledit coeur absorbant (12).

2. Article absorbant selon la revendication 1, **caractérisé en ce que** chaque partie de bord longitudinal (18, 20) est profilée.

3. Article absorbant selon la revendication 1, **caractérisé en ce que** la surface supérieure (14) est profilée.

4. Article absorbant selon la revendication 2 ou 3, **caractérisé en ce que** les parties profilées sont créées par compression de rainures de la feuille supérieure (36), et **en ce que** les premiers moyens d'espacement (60) sont constitués par les parties non comprimées de la feuille supérieure (36).

5. Article absorbant selon la revendication 2 ou 3, **caractérisé en ce que** les parties profilées sont rendues profilées par compression de rainures de la partie supérieure du coeur absorbant (12), et **en ce que** les premiers moyens d'espacement (60) sont constitués par les parties non comprimées du coeur absorbant (12).

6. Article absorbant selon la revendication 2 ou 3, **caractérisé en ce que** les parties profilées sont rendues profilées par compression de rainures à la fois de la feuille supérieure (36) et de la partie supérieure du coeur absorbant (12), et **en ce que** les premiers moyens d'espacement (60) sont constitués par les parties non comprimées de la feuille supérieure (36) et de la partie supérieure du coeur absorbant (12).

7. Article absorbant selon la revendication 2 ou 3, **caractérisé en ce que** les parties profilées sont rendues profilées par un matériau de distance sous la forme de cylindres, qui sont placés dans la feuille supérieure (36) de chaque partie profilée, dans une direction allant du centre de la serviette hygiénique (10) vers les côtés longitudinaux de la serviette hygiénique (10), et **en ce que** les premiers moyens d'espacement (60) sont constitués du matériau de distance.

8. Article absorbant selon la revendication 2 ou 3, **caractérisé en ce que** les parties profilées sont rendues profilées par un matériau de distance sous la forme de cylindres, qui sont placés dans la partie supérieure du coeur absorbant (12) de chaque partie profilée, dans une direction allant du centre de la serviette hygiénique (10) vers les côtés longitudinaux de la serviette hygiénique (10), et **en ce que** les premiers moyens d'espacement (60) sont constitués du matériau de distance.

9. Article absorbant selon l'une quelconque des revendications précédentes, **caractérisé en ce que** les barrières anti-fuite (68, 70) sont constituées de bandes formant barrières (46, 48), chaque bande recouvrant une partie de bord longitudinal (18, 20) respective et formant des poches de retenue de liquide le long de chaque partie de bord longitudinal, une paire d'éléments élastiques longitudinaux (50, 52) est placée le long desdites bandes formant barrières (46, 48) qui sont placées le long de chaque partie de bord longitudinal (18, 20) dudit coeur absorbant (12) au moins dans ladite partie centrale (34) du coeur absorbant.

10. Article absorbant selon la revendication 9, **caractérisé en ce que** les éléments élastiques (50, 52) se présentent sous la forme d'une chaîne de cordons, et **en ce que** les cordons définissent un deuxième moyen d'espacement.

11. Article absorbant selon la revendication 9, **caractérisé en ce que** les éléments élastiques (50, 52) se présentent sous la forme d'une chaîne de cylindres courts ayant une longueur inférieure au diamètre de cylindre, et **en ce que** les cylindres courts définissent des deuxièmes moyens d'espacement.

12. Article absorbant selon la revendication 9, **caractérisé en ce que** les éléments élastiques (50, 52) se présentent sous la forme d'une chaîne de cylindres longs ayant une longueur supérieure au diamètre de cylindre, et **en ce que** les cylindres longs définissent des deuxièmes moyens d'espacement.

13. Article absorbant selon la revendication 9, **caractérisé en ce qu'**un matériau de distance sous la forme de cylindres longs est placé à l'intérieur des bandes formant barrières (46, 48), dans une direction allant du centre de la serviette hygiénique (10) vers les côtés longitudinaux de la serviette hygiénique (10).

14. Article absorbant selon l'une quelconque des revendications précédentes, **caractérisé en ce que** les bandes formant barrières (46, 48) sont principalement hydrophobes.

15. Article absorbant selon l'une quelconque des revendications précédentes, **caractérisé en ce que** lesdits éléments élastiques latéraux (50, 52) se présentent sous la forme d'un rembourrage tridimensionnel en matériau plastique fibreux non tissé ou d'un plastique expansé.

16. Article absorbant selon l'une quelconque des revendications 1 à 8, **caractérisé en ce qu'**une feuille imperméable aux liquides (38) recouvrant lesdites parties de bords longitudinaux (18, 20) du coeur absorbant (12) et **en ce que** les barrières anti-fuite (68, 70) sont constituées d'une bande de matériau résilient (40) qui est placée entre ladite feuille imperméable aux liquides (38) et ledit coeur absorbant (12) le long de chaque partie de bord longitudinal (18, 20) du coeur absorbant au moins dans ladite partie médiane (34) du coeur absorbant pour augmenter de ce fait la résistance à la flexion de l'article.

17. Article absorbant selon la revendication 16, **caractérisé en ce que** ladite bande de matériau résilient (40) est un rembourrage tridimensionnel en matériau plastique fibreux non tissé ou un plastique expansé.

18. Article absorbant selon la revendication 16 ou 17, **caractérisé en ce que** ladite bande de matériau résilient (40) est placée entre ladite feuille imperméable aux liquides (38) et ladite surface inférieure (16) dudit coeur absorbant (12).

19. Article absorbant selon l'une quelconque des revendications 16 à 18, **caractérisé en ce qu'**il comprend une paire de feuilles imperméables aux liquides (38), chaque feuille recouvrant une partie de bord longitudinal (18, 20) respective et formant des poches de retenue de liquide le long de chaque partie de bord longitudinal (18, 20).

20. Article absorbant selon l'une quelconque des revendications précédentes, **caractérisé en ce que** le côté de ladite bande de matériau résilient (40) qui fait face au côté supérieur du coeur absorbant (12) est rendue profilée par compression de rainures.

21. Article absorbant selon la revendication 20, **caractérisé en ce que** les parties profilées non comprimées de la bande de matériau résilient (40) définissent des troisièmes moyens d'espacement (78).

22. Article absorbant selon la revendication 19, **caractérisé en ce qu'**il comprend une feuille inférieure (42) imperméable aux liquides s'étendant sur ladite surface inférieure (16) du coeur absorbant, ladite feuille inférieure (42) et ladite feuille supérieure (36) étant reliées pour former une marge périphérique (44) autour dudit coeur absorbant afin d'enfermer lesdites poches de retenue de liquide le long desdites parties de bord.

23. Article absorbant selon l'une quelconque des revendications précédentes, **caractérisé en ce que** lesdites poches s'étendent sur toute la longueur du coeur absorbant.

24. Article absorbant selon l'une quelconque des revendications précédentes, **caractérisé en ce que** lesdites poches s'étendent le long de la partie centrale (34) du coeur absorbant (12).

25. Article absorbant selon l'une quelconque des revendications précédentes, **caractérisé en ce que** la partie centrale (34) du coeur absorbant comprend entre 20 % et 60 %, de préférence entre 30 et 45 %, et de façon encore plus préférée environ un tiers, de la longueur du coeur absorbant (12).

26. Article absorbant selon l'une quelconque des revendications précédentes, **caractérisé en ce que** lesdites barrières anti-fuite (68, 70) comportent chacune une extension transversalement d'un bord à l'autre du coeur absorbant (12), ladite extension représentant entre 5 % et 25 % de la largeur totale de la serviette hygiénique dans la zone de mouillage.

27. Article absorbant selon l'une quelconque des revendications précédentes, **caractérisé en ce que** l'épaisseur des premiers, deuxièmes et troisièmes moyens d'espacement est d'au moins 1 mm.
